# FASCICULE DE BREVET EUROPEEN

(11) **EP 0 966 956 B1**
(45) Date de publication et mention de la délivrance du brevet: **31.01.2001**
(21) Numéro de dépôt: 99401415.7
(22) Date de dépôt: 10.06.1999
(51) Int. Cl.: A61K 7/50

(54) **Composition déformable ou malléable chauffante solide à hydrater pour le soin ou le nettoyage de la peau**
Feste form- oder knetbare Zusammensetzung erwärmbar durch Befeuchtung zur Pflege und Reinigung der Haut
Solid deformable or malleable composition heating when hydrated for care or cleaning of the skin

(30) Priorité: 15.06.1998 FR 9807518
(43) Date de publication de la demande: 29.12.1999
(73) Titulaire: L'OREAL, 75008 Paris (FR)
(72) Inventeur: Daubige, Thérèse, 77480 Mousseaux les Bray (FR); Roulier, Véronique, 75010 Paris (FR)
(74) Mandataire: Rasson, Catherine

(56) Documents cités:
- EP-A- 0 027 730
- EP-A- 0 692 240
- EP-A- 0 697 207
- EP-A- 0 745 378
- FR-A- 2 722 101

## Description

La présente invention a pour objet une composition anhydre, chauffante et moussante, pour le nettoyage en profondeur et/ou le soin de la peau ayant un aspect de solide déformable. Cette composition peut être appliquée aussi bien sur le visage que sur le corps d'être humain et est d'une très grande douceur. Cette composition de toucher sec doit être hydratée avant usage.

L'invention se rapporte aussi à un procédé cosmétique de nettoyage et/ ou de soin de la peau.

Les compositions nettoyantes pour la peau se présentent habituellement sous forme de pains solides comme les savons ou sous forme de liquides plus ou moins visqueux.

Pour se nettoyer avec un savon, les consommateurs se servent en général du savon entier. Or, ce savon a tendance à se ramollir au fur et à mesure de ses utilisations, du fait de son contact avec l'eau, et à mal vieillir. En outre, il est fréquent qu'il se casse et que les consommateurs se retrouvent avec des petits bouts de savon difficiles à utiliser. Par ailleurs, un savon mouillé est généralement glissant, ce qui rend son emploi malaisé, notamment pour les jeunes enfants. De ce fait, il est devenu courant d'utiliser des nettoyants liquides à la place de savons. Malheureusement, plus les compositions sont liquides, plus il est difficile de les doser, notamment parce qu'elles ont tendance à s'échapper entre les doigts, et plus elles ont tendance à s'échapper de leur conditionnement, ce qui peut être très gênant lorsqu'elles viennent au contact de vêtement, lors de déplacement.

Il est aussi connu d'utiliser des masques nettoyants pour le nettoyage en profondeur du visage. Ces masques se présentent généralement sous forme de gel ou crème à appliquer en couche mince sur la peau, contenant des poudres capables d'absorber les corps gras produits par la peau comme le sébum. Ces masques peuvent éventuellement contenir des actifs cosmétiques ou dermatologiques de la peau pour parfaire le nettoyage et/ou apporter du bien-être à la peau. De tels masques sont notamment décrits dans les documents US-A-5,690,945 et WO-A-86/05394. Ces masques sont souvent lourds à porter, peu confortables (tiraillement) et le nettoyage n'est pas toujours ressenti comme efficace. De plus, ces masques sont difficilement rinçables.

Par ailleurs, les utilisateurs recherchent, de plus en plus, de nouvelles textures et de nouveaux types de produits.

La présente invention a justement pour objet une nouvelle composition pour le nettoyage et/ou le soin de la peau, permettant notamment de remédier aux inconvénients mentionnés ci-dessus. Cette composition est une composition anhydre, chauffante et moussante, d'application aisée, d'une très grande légèreté et d'une grande douceur, donnant un effet de bien-être après application. Elle a, en outre, l'avantage de se rincer de façon remarquable et de présenter une texture tout à fait inhabituelle.

Ainsi, l'invention se rapporte à une composition anhydre, chauffante telle que définie dans la revendication 1.

On entend ici par « composition chauffante » une composition telle que l'utilisateur ressente un échauffement lors de l'application de la composition hydratée sur la peau. C'est une composition qui, en présence d'eau, peut élever la température du mélange de plusieurs degrés (un à dix degrés) de manière instantanée.

Cette composition anhydre est destinée plus spécialement au nettoyage et/ou soin du visage d'être humain. Elle se présente sous forme de solide déformable ou malléable, sec, ne tachant pas et ressemblant à de la guimauve (voir le document US-A-3,682,659 pour la consistance de la guimauve). Ce solide peut être modelé comme de la pâte à modeler pour enfants. Il peut être rompu facilement à la main afin de ne prélever que la quantité nécessaire de produit. En particulier, cette composition peut être conditionnée sous forme de monodose, particulièrement avantageuse d'un point de vue hygiénique, et par exemple sous forme de petits cubes, de billes ou de berlingots.

Grâce à cette texture solide, la composition de l'invention ne risque pas de s'échapper de son conditionnement notamment lors d'un transport. Par ailleurs cette composition est très facilement préhensible et ne s'écoule pas entre les doigts ; son dosage est beaucoup plus simple que celui des nettoyants liquides habituels et le problème d'usure des savons durs ne se pose pas. De plus, son application est aisée, légère et agréable. Par ailleurs, son stockage ne pose pas de problème et sa pollution par l'environnement et/ou la manipulation par le consommateur est relativement réduite, et en tout état de cause très inférieure à celle des produits comparables de l'art antérieur. En particulier, il n'est pas nécessaire d'introduire de conservateur pour assurer sa protection antimicrobienne.

La texture de solide déformable est principalement due à la poudre.

En vue d'obtenir un solide au toucher agréable et doux, il est préférable d'utiliser des particules ayant une granulométrie de 1 µm à 300 µm, par exemple de 5 µm à 200 µm et de préférence de 10 µm à 100 µm et mieux de 15 µm à 40 µm.

La grande douceur apportée par ces particules permet l'utilisation de la composition de l'invention par des personnes à peau sensible.

Grâce à la présence de l'agent susceptible de dégager de la chaleur, le nettoyage se fait en profondeur, par suite d'une ouverture des pores de la peau, facilitant l'excrétion des corps gras et des impuretés. Ces impuretés et corps gras sont au fur et à mesure de leur sortie de la peau absorbés par la poudre, sans toutefois dessécher et tirailler la peau.

Afin de conférer à la composition de l'invention un aspect aéré et léger, on utilise avantageusement des particules ayant une densité inférieure à 0,09 et mieux inférieure à 0,06 et encore mieux inférieure à 0,04.

En vue d'obtenir cette faible densité, on utilise avantageusement des particules creuses remplies d'un gaz. Ce gaz peut être de l'air, de l'azote, de l'isobutane, de l'isopentane, etc.

Selon une autre caractéristique avantageuse de l'invention, les particules se présentent sous forme de billes. Il est toutefois possible d'utiliser des particules ayant la forme de fibres.

Ces particules peuvent être réalisées en différents matériaux inertes ne réagissant pas chimiquement avec le liant cosmétiquement acceptable ; en particulier ces particules ne réagissent pas avec les huiles, les tensioactifs, et les différents autres constituants de la composition, comme les actifs.

La poudre à utilisation dans une composition selon l'invention, conférant la texture de solide déformable, a la particularité de se déliter facilement par simple dilution dans un solvant comme l'eau éventuellement chargée en sels ou oligo-éléments.

Comme critère de choix de la poudre, on peut réaliser le test suivant :
- ajout de particules déterminées dans de l'eau contenant un colorant classiquement utilisé dans le domaine nettoyant, tel que le sel disodique de bleu brillant FCF codifié dans le Color Index sous la référence Cl 42090, jusqu'à l'obtention d'une pâte colorée,
- versement d'une goutte d'eau sur la pâte.

Lorsque la pâte au point d'impact de la goutte d'eau est beaucoup plus claire que le reste de la pâte, cela signifie que les particules considérées sont appropriées pour texturer la composition. Inversement, lorsque la pâte au point d'impact ne s'est pas décolorée, les particules considérées ne sont nullement appropriées.

Les particules inertes sont avantageusement réalisées en matériaux thermoplastiques comme les polymères ou copolymères d'acrylonitrile, de chlorure de vinylidène, de chlorure de vinyle et/ou de monomère acrylique ou styrénique, éventuellement expansés. Le monomère acrylique est par exemple un acrylate ou méthacrylate de méthyle ou d'éthyle. Le monomère styrénique est par exemple l'α-méthyl-styrène ou le styrène.

De préférence, les particules sont des particules creuses déformables de copolymère expansé de chlorure de vinylidène et d'acrylonitrile ou de chlorure de vinylidène, d'acrylonitrile et de méthacrylate. On peut par exemple utiliser un copolymère contenant de 0 % à 60 % de motifs dérivés du chlorure de vinylidène, de 20 % à 90 % de motifs dérivés d'acrylonitrile et de 0 % à 50 % de motifs dérivés d'un monomère acrylique ou styrénique, la somme des pourcentages (en poids) étant égale à 100. Ces particules se présentent notamment à l'état sec ou hydraté et peuvent être obtenues, par exemple, selon les procédés décrits dans les brevets et demandes de brevet EP-A-56 219, EP-A-348 572, EP-A-320 473, EP-A-112 807 et US-A-3,615,972.

Ces particules creuses peuvent être par exemple celles formées d'un terpolymère de chlorure de vinylidène, d'acrylonitrile et de méthacrylate, vendues sous la marque Expancel par la société Nobel Casco sous les références 551 DE 12 (granulométrie d'environ 12 µm et masse volumique 40 kg/m³), 551 DE 20 (granulométrie d'environ 30 µm et masse volumique 65 kg/m³), 551 DE 50 (granulométrie d'environ 40 µm). On peut aussi citer les microsphères formées du même terpolymère expansé à l'état sec ayant une granulométrie d'environ 18 µm et une masse volumique d'environ 60 à 80 kg/m³, appelées ci-dessous EL 23, ou ayant une granulométrie d'environ 34 µm et une masse volumique d'environ 20 kg/m³, appelées ci-dessous EL 43, ou ayant une granulométrie d'environ 150 µm, appelées ci-dessous EL 55.

En revanche, les particules d'amidon de maïs, de silice pyrogénée, de polyéthylène, de polyuréthanne ou de polyester non expansé ne permettent pas d'obtenir une composition solide qui s'élimine bien de la peau lors du rinçage.

L'obtention ou non d'un solide déformable est liée à la quantité de poudre ou agent structurant, utilisée dans la composition ; au-dessus d'une certaine quantité de particules, appelée charge pigmentaire volumique critique et notée CPVC, on note une augmentation brusque de la viscosité du milieu. La CPVC est fonction du milieu et de la nature des particules ; elle doit donc être déterminée à chaque fois. Sa détermination ne pose aucun problème pour l'homme du métier. On peut, par exemple, utiliser la méthode officielle ASTM pour déterminer la CPVC.

En pratique la poudre structurante représente jusqu'à 80 % en volume de la composition, dont avantageusement 60 % représentent des particules ayant une densité inférieure à 1. Dans ces conditions, le liant représente jusqu'à 20 % en volume de la composition. En particulier, les particules de densité inférieure à 1 représentent de 2 à 20 % et mieux de 3 à 7 % du poids total de la composition.

L'invention a encore pour objet l'utilisation cosmétique de la composition définie précédemment pour le nettoyage et/ou le soin de la peau d'êtres humains.

Ainsi, l'invention a encore pour objet un procédé cosmétique de nettoyage et/ou de soin de la peau, consistant à hydrater une composition telle que définie ci-dessus, à la faire mousser, à l'appliquer sur la peau, et enfin à rincer la peau.

La composition de l'invention contient, outre les particules structurantes, une quantité efficace d'un agent susceptible de dégager de la chaleur, lors de l'hydratation de la composition. Cet agent est notamment choisi parmi les polyols ayant au moins 2 groupes hydroxyle et au moins 3 atomes de carbone, comme notamment la glycérine, la diglycérine, le propylène glycol, le butylène glycol, un polyéthylène glycol de poids moléculaire inférieur à 600 comme le PEG 400 vendu par la société BASF sous le nom Lutrol E400, les sucres tels que le sorbitol, leurs mélanges. Ce type d'agent présente la particularité de réagir chimiquement avec l'eau selon un processus exothermique. Pour que ce processus exothermique ait lieu, il est souhaitable que la composition soit exempte d'eau.

La quantité de cet agent doit être telle que l'utilisateur ressente effectivement un échauffement lors de l'application de la composition, après hydratation. En pratique, l'agent chauffant représente de 20 à 85 % du poids total de la composition et mieux de 33 à 67 %.

La composition selon l'invention contient un ou plusieurs tensioactifs nettoyants et/ou moussants, anhydres, qui peuvent être des tensioactifs non ioniques, anioniques, cationiques et/ou amphotères. Ils peuvent être utilisés en une quantité allant par exemple de 10 % à 60 % du poids total de la composition, et de préférence de 30 % à 60 %. Le ou les tensioactifs anhydres sont sous forme de poudre. Ils peuvent présenter une granulométrie allant de 5 à 50 µm et mieux de 10 à 20 µm.

Comme tensioactifs non ioniques utilisables dans l'invention, on peut citer par exemple les condensats d'oxydes d'alkylène et d'alkyl phénols comme l'octyl phénol éthoxylé tel que celui vendu sous le nom Triton X45 par la société Rohm & Haas, les condensats d'oxyde d'éthylène, d'oxyde de propylène et d'éthylène diamine, les alkylpolyglucosides, les éthers d'alcool gras et de polyols comme par exemple le Polyglyceryl-3 hydroxylauryl Ether (nom CTFA) vendu sous la dénomination Chimexane NF par la Société Chimex, leurs mélanges.

Comme tensioactifs anioniques, on peut citer par exemple les polyalkylènes glycols éther d'alcools gras, les taurates, les acyl lactylates comme le stéaroyl lactylate de sodium (par exemple Pationic SSL vendu par la société Maprecos), les alkyl sulfates comme le lauryl sulfate de sodium (Sipon LCS vendu par la société Henkel), les alkyl sulfates de glycéryle comme le cocoglycéryl sulfate de sodium (vendu par la société Nikko sous le nom Nikkol SGC-80N), les alkyl sulfates polyoxyéthylénés, les alkyl éthers sulfates comme le lauryl éther sulfate de monoéthanolamine, les alkyl éthers carboxylates, les monoalkyl ou dialkyl phosphates comme le monohexyl-2-décyl phosphate d'argine (C6C10 MAP-1-ARG vendu par la société Kao Chemicals), les alkyl phosphate éthoxylés, les N-acyl sarcosinates comme le myristoyl sarcosinate de sodium (par exemple Nikkol Sarcosinate MN vendu par la société Nikko), les N-acyl glutamates comme le lauroyl glutamate de sodium (par exemple Amisoft LS11 vendu par la société Ajinomoto), les acyl iséthionates comme le cocoyl iséthionate de sodium vendu notamment par la société Jordan (Jordapon Cl), les polysorbates, les succinamates, les savons comme le laurate, myristate, palmitate ou stéarate de potassium, leurs mélanges.

Comme tensioactifs amphotères ou zwitterions, on peut citer par exemple les bétaïnes et les dérivés de bétaïnes, les sultaïnes et les dérivés de sultaïnes, les dérivés imidazolinium, comme le disodium cocoamphodiacétate, leurs mélanges.

Comme tensioactifs cationiques utilisables dans l'invention, on peut citer les dérivés de pyrrolidone carboxylate comme le PCA éthyl cocoyl arginate (Cation CAE vendu par la société Ajinomoto).

La composition selon l'invention peut, en outre, contenir une ou plusieurs huiles qui peuvent être utilisées en une quantité allant de 0 % à 30 % en poids, et de préférence de 0 % à 10 % du poids total de la composition. Ces huiles font partie intégrante du liant.

Les huiles utilisables dans la composition selon l'invention peuvent être choisies parmi les huiles minérales comme l'huile de paraffine et l'huile de vaseline ; les huiles d'origine animale comme le perhydrosqualène ; les huiles d'origine végétale telles que l'huile d'amande douce, d'avocat, de ricin, d'olive, de jojoba, de sésame. d'arachide, de pépins de raisin, de colza, de coprah, de noisettes, de palme, de noyau d'abricot, de calophyllum, de son de riz, de germes de maïs, de germes de blé, de soja, de tournesol, de carthame, de passiflore, de seigle et le beurre de karité et sa fraction liquide ; les huiles de synthèse telles que les esters gras comme le myristate de butyle ou d'isopropyle, le stéarate d'hexadécyle, d'isopropyle, d'octyle ou d'isodécyle, l'oléate de décyle, le laurate d'héxyle, le dicaprylate de propylène glycol, les esters dérivés d'acide lanolique comme le lanolate d'isopropyle et le lanolate d'isocétyle, les isoparaffines, les acétylglycérides, les octanoates d'alcools et de polyalcools tels que ceux de glycol et de glycérol, les ricinoléates d'alcools et de polyalcools, les triglycérides d'acides gras ; les huiles de silicone telles que les cyclométhicones, les polydiméthylsiloxanes volatiles et/ou non volatiles ou encore les phényldiméthylsiloxanes ; leurs mélanges.

La composition peut, en outre, contenir un ou plusieurs autres ingrédients lipophiles, classiquement utilisés dans les compositions nettoyantes et/ou de soin. Ces ingrédients sont, notamment, des filtres, des parfums, des conservateurs, des antioxydants, des agents régulateurs de pH, des séquestrants, des charges, des colorants, des actifs cosmétiques ou dermatologiques, ou leurs mélanges. Ces adjuvants sont utilisés dans les proportions habituelles des compositions nettoyantes et/ou de soin, et par exemple de 0,01 à 10 % du poids total de la composition. Ces adjuvants doivent être de nature et utilisés en quantité telles qu'ils ne perturbent pas les propriétés recherchées pour la composition de l'invention.

Comme actifs utilisables dans l'invention, on peut citer des antibactériens comme l'octopirox et le triclosan, des agents kératolytiques comme l'acide salicylique, des huiles essentielles, des vitamines lipophiles.

La composition selon l'invention peut être préparée par tout moyen connu de l'homme du métier, et en particulier par simple mélange des différents constituants et moulage dans un moule adéquat. Cependant, de façon avantageuse, elle est préparée par mélange suivi d'un malaxage puis d'une extrusion dans un extrudeur, de préférence un extrudeur bi-vis tel que ceux décrits dans les documents EP-A-605284 et FR-A-2,715,306, et dans lequel les deux vis tournent dans le même sens. On peut aussi utiliser le procédé décrit dans le document EP-A-651991.

L'invention a encore pour objet un procédé de préparation de la composition ci-dessus, consistant à introduire puis malaxer les différents constituants de la composition dans un extrudeur et à mettre en forme le mélange obtenu.

Les différents constituants de la composition sont introduits, à l'entrée de l'extrudeur bi-vis, dans la zone d'alimentation à température ambiante, de préférence environ 20°C. De préférence, on introduit les constituants solides en tête de l'extrudeur puis les constituants liquides sont introduits latéralement. Le tout est malaxé dans diverses zones de l'extrudeur maintenues à une température allant, préférentiellement, de 15 à 25°C ; la masse obtenue est ensuite transportée vers la sortie de l'extrudeur et extrudée au travers d'une filière. La vitesse de rotation des vis est de l'ordre de 400 à 1000 tours/minute, de préférence comprise entre 550 et 650 tours/minute.

La masse extrudée sort de la filière sous la forme de boudins de diamètre donné selon la filière utilisée, pouvant être ensuite découpés et mis en forme, notamment, sous forme de bâton ou de pain solide. D'autres formes peuvent bien entendu être réalisées en choisissant des filières appropriées et des dispositifs de mise en forme des produits finaux, adaptés à la forme recherchée.

La masse extrudée peut également être déshydratée et/ou broyée et/ou compactée. La déshydratation est avantageusement utilisée lorsque les constituants de la composition sont introduits sous forme de solution ou de dispersion en milieu aqueux.

Du fait que la totalité du procédé d'extrusion est réalisée à température ambiante, de l'ordre de 20-25°C, il est possible d'utiliser des ingrédients sensibles à la chaleur, du type vitamines ou huiles volatiles.

D'autre part, les ingrédients sensibles à la chaleur peuvent être introduits dans n'importe quelle zone de l'extrudeur (en tête, au milieu ou en final) puisqu'aucune détérioration due à la chaleur n'est à craindre. Ceci est en particulier avantageux pour l'introduction des agents structurant du type Expancel.

Il est également possible d'effectuer une partie de l'extrusion sous gaz inerte (azote par exemple).

Les exemples ci-après sont donnés à titre illustratif et non limitatif en vue de mieux faire ressortir les caractéristiques de l'invention. Les quantités y sont données en % en poids.

### Exemple 1 : Composition chauffante et moussante pour peaux grasses à tendance acnéique

- Cocoyl iséthionate de sodium (Jordapon Cl vendu par la société Jordan) 44 %
- Glycérine 50 %
- Conservateur qs
- Octopirox 0,1 %
- Parfum qs
- Acide salicylique 0,5 %
- Expancel 551 DE 20 5,0 %

Le mode opératoire consiste à introduire à froid le tensioactif et la glycérine en tête d'un extrudeur bi-vis, puis à introduire latéralement dans les différents étages de l'extrudeur les actifs, les conservateurs, le parfum et, au final, l'Expancel. Le mélange est extrudé à froid jusqu'à obtention d'une pâte homogène, qui est ensuite mise en forme dans une filière appropriée pour obtenir un boudin que l'on découpe en bâton.

Le produit obtenu est une pâte blanche, non collante, facilement modelable, facilement mouillable ou hydratable, d'application et d'élimination faciles, douce au toucher et ayant un bon pouvoir moussant.

Ce produit peut alors être hydraté, puis malaxé jusqu'à ce qu'il mousse notamment dans le creux de la main. La mousse obtenue est alors appliquée en couche mince sur la peau du visage. La quantité d'eau peut représenter de 6 à 10 fois en volume celle de l'échantillon prélevé pour le nettoyage.

Dès l'application de la mousse sur le visage, on ressent un léger dégagement de chaleur, assurant l'ouverture des pores de la peau et la libération des impuretés. Ensuite, les actifs anti-acné pénètrent dans la peau pour la nettoyer en profondeur et la désinfecter en vue d'empêcher la formation de bouton. L'élimination de la composition se fait aisément avec de l'eau.

### Exemple 2 : Composition chauffante et moussante pour tout type de peaux

- Cocoyl iséthionate de sodium (Jordapon Cl vendu par la société Jordan) 55 %
- Expancel 551 DE 20 5 %
- PEG 400 40 %

Le mode opératoire est le même que pour l'exemple 1 et on obtient une pâte ayant des propriétés analogues.

### Exemple 3 : Composition chauffante et moussante pour peaux sensibles

- Cocoyl iséthionate de sodium (Jordapon Cl vendu par la société Jordan) 36 %
- Glycérine 57 %
- Huile d'amande douce 1 %
- Microsphères EL 23 6 %

Le mode opératoire est le même que pour l'exemple 1 et on obtient une pâte ayant des propriétés analogues.

## Revendications

1. Composition anhydre chauffante se présentant sous forme de solide déformable ou malléable, à hydrater pour le nettoyage et/ou le soin de la peau, contenant une poudre et un liant cosmétiquement acceptable, la poudre comprenant des particules solides de polymère expansé, le liant contenant une quantité efficace d'un agent susceptible de dégager de la chaleur, lors de l'hydratation, la composition contenant au moins un tensioactif moussant et/ou nettoyant anhydre sous forme de poudre.

2. Composition selon la revendication 1, caractérisée en ce que les particules ont une granulométrie de 1 µm à 300 µm.

3. Composition selon la revendication 1 ou 2, caractérisée en ce que les particules ont une granulométrie de 10 µm à 100 µm.

4. Composition selon l'une des revendications précédentes, caractérisée en ce que les particules ont une densité inférieure à 0,09.

5. Composition selon l'une des revendications précédentes, caractérisée en ce que les particules ont une densité inférieure à 0,04.

6. Composition selon l'une des revendications précédentes, caractérisée en ce que les particules sont présentes en une concentration au moins égale à la charge pigmentaire volumique critique.

7. Composition selon l'une des revendications précédentes, caractérisée en ce que les particules sont creuses.

8. Composition selon l'une des revendications précédentes, caractérisée en ce que les particules sont en matériau thermoplastique.

9. Composition selon l'une des revendications précédentes, caractérisée en ce que les particules sont réalisées en un matériau choisi parmi les polymères et copolymères de chlorure de vinylidène, de chlorure de vinyle, d'acrylonitrile et/ou d'un monomère acrylique ou styrénique.

10. Composition selon l'une des revendications précédentes, caractérisée en ce que les particules sont des particules creuses de copolymère de chlorure de vinylidène, d'acrylonitrile et/ou d'un monomère acrylique ou styrénique.

11. Composition selon l'une des revendications précédentes, caractérisée en ce que l'agent susceptible de dégager de la chaleur est choisi parmi les polyols.

12. Composition selon l'une des revendications précédentes, caractérisée en ce que l'agent susceptible de dégager de la chaleur est choisi parmi la glycérine, la diglycérine, les polyéthylènes glycol, le propylène ou butylène glycol, les sucres et leurs mélanges.

13. Composition selon l'une des revendications précédentes, caractérisée en ce que l'agent susceptible de dégager de la chaleur est présent en une quantité allant de 20 à 85 % du poids total de la composition.

14. Composition selon l'une des revendications précédentes, caractérisée en ce que l'agent susceptible de dégager de la chaleur est présent en une quantité allant de 33 à 67 % du poids total de la composition.

15. Composition selon l'une des revendications précédentes, caractérisée en ce que le tensioactif est le cocoyl iséthionate de sodium.

16. Composition selon l'une des revendications précédentes, caractérisée en ce que le tensioactif est présent à raison de 10 à 60 % du poids total de la composition.

17. Composition selon l'une des revendications précédentes, caractérisée en ce que le tensioactif est présent à raison de 30 % à 60 % du poids total de la composition .

18. Composition selon l'une des revendications précédentes, caractérisée en ce qu'elle contient, en outre, au moins un ingrédient lipophile choisi parmi les filtres, les parfums, les conservateurs, les antioxydants, les agents régulateurs de pH, les séquestrants, les charges, les colorants, les actifs cosmétiques ou dermatologiques, leurs mélanges.

19. Composition selon l'une des revendications précédentes, caractérisée en ce qu'elle est obtenue par extrusion à froid.

20. Utilisation cosmétique de la composition selon l'une des revendications précédentes pour le nettoyage et/ou le soin de la peau d'êtres humains.

21. Procédé cosmétique de nettoyage et/ou de soin de la peau, consistant à hydrater une composition selon l'une des revendications 1 à 20, à la faire mousser, puis à l'appliquer sur la peau et enfin à rincer la peau.

22. Procédé de préparation de la composition selon l'une des revendications 1 à 20, consistant à introduire puis malaxer les différents constituants de la composition dans un extrudeur et à mettre en forme le mélange obtenu.

23. Procédé selon la revendication 22, caractérisé en ce que les différents constituants de la composition sont introduits, à l'entrée d'un extrudeur bi-vis, dans la zone d'alimentation à température ambiante, en ce que le tout est malaxé dans diverses zones de l'extrudeur et en ce que la masse obtenue est ensuite transportée vers la sortie de l'extrudeur et extrudée au travers d'une filière.

24. Procédé selon la revendication 22 ou 23, caractérisé en ce que les constituants solides sont introduits en tête de l'extrudeur puis les constituants liquides sont introduits latéralement.

## Patentansprüche

1. Wasserfreie, wänneentwickelnde Zusammensetzung für die Reinigung und/oder Pflege der Haut, die angefeuchtet werden soll, wie ein deformierbarer oder modellierfähiger Feststoff aussieht und ein Pulver und ein kosmetisch akzeptables Bindemittel enthält, wobei das Pulver feste Partikel aus einem expandierten Polymer enthält und das Bindemittel eine wirksame Menge eine Stoffes enthält, der bei der Hydratisierung Wärme entwickeln kann, und wobei die Zusammensetzung mindestens einen wasserfreien, schäumenden und/oder reinigenden grenzflächenaktiven Stoff in Form eines Pulvers enthält.

2. Zusammensetzung nach Anspruch 1, dadurch gekennzeichnet, daß die Partikel eine Partikelgröße von 1 µm bis 300 µm aufweisen.

3. Zusammensetzung nach Anspruch 1 oder 2, dadurch gekennzeichnet, daß die Partikel eine Partikelgröße von 10 µm bis 100 µm aufweisen.

4. Zusammensetzung nach einem der vorhergehenden Ansprüche, dadurch gekennzeichnet, daß die Partikel eine Dichte unter 0,09 aufweisen.

5. Zusammensetzung nach einem der vorhergehenden Ansprüche, dadurch gekennzeichnet, daß die Partikel eine Dichte unter 0,04 aufweisen.

6. Zusammensetzung nach einem der vorhergehenden Ansprüche, dadurch gekennzeichnet, daß die Partikel in einer Konzentration vorliegen, die mindestens der kritischen, volumenbezogenen Pigmentkonzentration entspricht.

7. Zusammensetzung nach einem der vorhergehenden Ansprüche, dadurch gekennzeichnet, daß die Partikel hohl sind.

8. Zusammensetzung nach einem der vorhergehenden Ansprüche, dadurch gekennzeichnet, daß die Partikel aus einem thermoplastischen Material bestehen.

9. Zusammensetzung nach einem der vorhergehenden Ansprüche, dadurch gekennzeichnet, daß die Partikel aus einem Material hergestellt sind, das unter den Polymeren und Copolymeren von Vinylidenchlorid, Vinylchlorid, Acrylnitril und/oder einem Acryl- oder Styrolmonomer ausgewählt ist.

10. Zusammensetzung nach einem der vorhergehenden Ansprüche, dadurch gekennzeichnet, daß die Partikel expandierte Hohlpartikel eines Copolymers von Vinylidenchlorid, Acrylnitril und/oder einem Acryl- oder Styrolmonomer sind.

11. Zusammensetzung nach einem der vorhergehenden Ansprüche, dadurch gekennzeichnet, daß der Stoff, der Wärme entwickeln kann, unter den Polyolen ausgewählt ist.

12. Zusammensetzung nach einem der vorhergehenden Ansprüche, dadurch gekennzeichnet, daß der Stoff, der Wärme entwickeln kann, unter Glycerin, Diglycerin, Polyethylenglykolen, Propylenglykol, Butylenglykol, Zuckern und deren Gemischen ausgewählt ist.

13. Zusammensetzung nach einem der vorhergehenden Ansprüche, dadurch gekennzeichnet, daß der Stoff, der Wärme entwickeln kann, in einem Mengenanteil von 20 bis 85 % des Gesamtgewichts der Zusammensetzung vorliegt.

14. Zusammensetzung nach einem der vorhergehenden Ansprüche, dadurch gekennzeichnet, daß der Stoff, der Wärme entwickeln kann, in einem Mengenanteil von 33 bis 67 % des Gesamtgewichts der Zusammensetzung vorliegt.

15. Zusammensetzung nach einem der vorhergehenden Ansprüche, dadurch gekennzeichnet, daß der grenzflächenaktive Stoff das Natriumcocoylisethionat ist.

16. Zusammensetzung nach einem der vorhergehenden Ansprüche, dadurch gekennzeichnet, daß der grenzflächenaktive Stoff in einem Mengenanteil von 10 bis 60 % des Gesamtgewichts der Zusammensetzung vorliegt.

17. Zusammensetzung nach einem der vorhergehenden Ansprüche, dadurch gekennzeichnet, daß der grenzflächenaktive Stoff in einem Mengenanteil von 30 bis 60 % des Gesamtgewichts der Zusammensetzung vorliegt.

18. Zusammensetzung nach einem der vorhergehenden Ansprüche, dadurch gekennzeichnet, daß sie außerdem mindestens einen lipophilen Bestandteil enthält, der unter Filtern, Parfüms, Konservierungsmitteln, Antioxidantien, den pH-Wert regulierenden Mitteln, Maskierungsmitteln, Füllstoffen, Farbstoffen, kosmetischen oder dermatologischen Wirkstoffen und ihren Gemischen ausgewählt ist.

19. Zusammensetzung nach einem der vorhergehenden Ansprüche, dadurch gekennzeichnet, daß sie durch eine in der Kälte durchgeführte Extrusion hergestellt ist.

20. Kosmetische Verwendung der Zusammensetzung nach einem der vorhergehenden Ansprüche für die Reinigung und/oder Pflege der menschlichen Haut.

21. Kosmetisches Verfahren zur Reinigung und/oder Pflege der Haut, das darin besteht, eine Zusammensetzung nach einem der Ansprüche 1 bis 20 zu befeuchten, zum Schäumen zu bringen, auf die Haut aufzutragen und dann die Haut abzuspülen.

22. Verfahren zur Herstellung der Zusammensetzung nach einem der Ansprüche 1 bis 20, das darin besteht, die verschieden Bestandteile der Zusammensetzung in einem Extruder zu geben, anschließend zu kneten und das gebildete Gemisch dann zu formen.

23. Verfahren nach Anspruch 22, dadurch gekennzeichnet, daß die verschiedenen Bestandteile der Zusammensetzung bei Raumtemperatur am Kopf eines Doppelschneckenextruders in die Materialzufuhrzone gegeben werden, das Ganze in den verschiedenen Bereichen des Extruders geknetet wird und die erhaltene Masse zum Auslaß des Extruders gefördert und durch eine Düse extrudiert wird.

24. Verfahren nach Anspruch 22 oder 23, dadurch gekennzeichnet, daß die festen Bestandteile bevorzugt am Kopf des Extruders zugegeben und die flüssigen Bestandteile seitlich zugeführt werden.

## Claims

1. Warming anhydrous composition which is provided in the form of a deformable or malleable solid, to be hydrated for cleaning and/or caring for the skin, comprising a powder and a binder of cosmetically acceptable type, the powder comprising solid particles of expanded polymer, the binder comprising an effective amount of an agent capable of giving off heat during hydration and the composition comprising at least one foaming and/or cleaning anhydrous surfactant in the powder form.

2. Composition according to Claim 1, characterized in that the particles have a particle size of 1 µm to 300 µm.

3. Composition according to Claim 1 or 2, characterized in that the particles have a particle size of 10 µm to 100 µm.

4. Composition according to one of the preceding claims, characterized in that the particles have a density of less than 0.09.

5. Composition according to one of the preceding claims, characterized in that the particles have a density of less than 0.04.

6. Composition according to one of the preceding claims, characterized in that the particles are present at a concentration at least equal to the critical pigment volume concentration.

7. Composition according to one of the preceding claims, characterized in that the particles are hollow.

8. Composition according to one of the preceding claims, characterized in that the particles are made of a thermoplastic material.

9. Composition according to one of the preceding claims, characterized in that the particles are prepared from a material chosen from polymers and copolymers of vinylidene chloride, of vinyl chloride, of acrylonitrile and/or of an acrylic or styrene monomer.

10. Composition according to one of the preceding claims, characterized in that the particles are hollow particles of copolymer of vinylidene chloride, of acrylonitrile and/or of an acrylic or styrene monomer.

11. Composition according to one of the preceding claims, characterized in that the agent capable of giving off heat is chosen from polyols.

12. Composition according to one of the preceding claims, characterized in that the agent capable of giving off heat is chosen from glycerol, diglycerol, polyethylene glycols, propylene or butylene glycol, sugars and their mixtures.

13. Composition according to one of the preceding claims, characterized in that the agent capable of giving off heat is present in an amount ranging from 20 to 85% of the total weight of the composition.

14. Composition according to one of the preceding claims, characterized in that the agent capable of giving off heat is present in an amount ranging from 33 to 67% of the total weight of the composition.

15. Composition according to one of the preceding claims, characterized in that the surfactant is sodium cocoyl isethionate.

16. Composition according to one of the preceding claims, characterized in that the surfactant is present in a proportion of 10 to 60% of the total weight of the composition.

17. Composition according to one of the preceding claims, characterized in that the surfactant is present in a proportion of 30% to 60% of the total weight of the composition.

18. Composition according to one of the preceding claims, characterized in that it additionally comprises at least one lipophilic ingredient chosen from screening agents, fragrances, preservatives, antioxidants, pH-regulating agents, sequestering agents, fillers, dyes, cosmetic or dermatological active principles, or their mixtures.

19. Composition according to one of the preceding claims, characterized in that it is obtained by extrusion under cold conditions.

20. Cosmetic use of the composition according to one of the preceding claims for cleaning and/or caring for human skin.

21. Cosmetic process for cleaning and/or caring for the skin, which consists in hydrating a composition according to one of Claims 1 to 20, in causing it to foam, in then applying it to the skin and, finally, in rinsing the skin.

22. Process for the preparation of the composition according to one of Claims 1 to 20, which consists in introducing the various constituents of the composition into an extruder, in then kneading them therein, and in shaping the mixture obtained.

23. Process according to Claim 22, characterized in that the various constituents of the composition are introduced at room temperature at the inlet of a twinscrew extruder in the feed region, in that the combined mixture is kneaded in various regions of the extruder and in that the mass obtained is subsequently transported towards the outlet of the extruder and extruded through a die.

24. Process according to Claim 22 or 23, characterized in that the solid constituents are introduced at the head of the extruder and then the liquid constituents are introduced laterally.
